# EUROPEAN PATENT APPLICATION

(11) **EP 4 516 924 A1**
(43) Date of publication of application: **05.03.2025**
(21) Application number: 23909692.8
(22) Date of filing: 08.11.2023
(51) Int. Cl.: C12Q 1/6844, C12Q 1/70, C12N 15/11, C12R 1/93

(54) **RECOMBINASE-MEDIATED FULL-FREEZE-DRYING CONSTANT-TEMPERATURE AMPLIFICATION SYSTEM, PREPARATION METHOD THEREFOR, AND USE THEREOF**

(30) Priority: 30.12.2022 CN 202211720270
(71) Applicant: Sansure Biotech Inc., Changsha, Hunan 410205 (CN)
(72) Inventor: DING, Pinghui, Changsha, Hunan 410205 (CN); CAO, Changzheng, Changsha, Hunan 410205 (CN); JI, Bozhi, Changsha, Hunan 410205 (CN); LIU, Jia, Changsha, Hunan 410205 (CN); DAI, Lizhong, Changsha, Hunan 410205 (CN)
(74) Representative: RGTH
(86) International application number: PCT/CN2023/130567
(87) International publication number: WO 2024/139760

(57) **Abstract**

Provided are a recombinase-mediated full-freeze-dried constant-temperature amplification system, a preparation method therefor, and use thereof. The full-freeze-dried constant-temperature amplification system includes two parts: a first part being an enzyme system, a second part being a primer, a probe and an activation system; the freeze-dried form of the enzyme system is freeze-dried powder, and that of the primer and the probe and the activation system are freeze-dried pellets. By preparing the enzyme system into freeze-dried powder, and mixing and preparing the activation system and the primer and the probe into freeze-dried pellets, the smoothness and homogeneity of liquid are improved, thereby avoiding the problem of unstable results due to high thickness of the whole system and thus difficult to disperse and homogenize during freeze-dried processes. Full-freeze-dried for RPA is achieved by easy operation of one-step sample loading. A kit including the full-freeze-dried constant-temperature amplification system has high sensitivity and specificity.

## Description

### CROSS-REFERENCE TO RELATED APPLICATIONS

This application claims the priority of Chinese patent application CN202211720270.0 filed on December 30, 2022, titled "Recombinase-mediated Full-freeze-drying Constant-temperature Amplification System, Preparation Method Therefor, and Use Thereof", which is incorporated herein by reference in its entirety.

### FIELD OF THE INVENTION

The application belongs to the field of molecular biological detection, and particularly relates to a recombinase-mediated full-freeze-dried constant-temperature amplification system and a preparation method therefor and use thereof.

### BACKGROUND OF THE INVENTION

Recombinase polymerase amplification (RPA) is known as a nucleic acid detection technology that can replace PCR. RPA technology mainly relies on three enzymes: recombinase that can bind to single-stranded nucleic acids (oligonucleotide primers), single-stranded DNA binding protein (SSB), and strand-displacing DNA polymerase. The mixture of these three enzymes is also active at room temperature, and the optimal reaction temperature is around 37-40°C. RPA has a fast reaction speed, short reaction time and strong specificity. Currently, RPA reagents can be stored in the form of freeze-dried powder or liquid. Prepackaging the freeze-dried RPA reagents can avoid errors in experimental results caused by repeated freezing and thawing, prevent the loss of high-viscosity components such as enzymes, and can be stored and transported at room temperature with a simple packaging form.

However, since the RPA system relies on the action of amplification enzymes and proteins, and the enzymes and proteins in the system can react at room temperature, the form and storage conditions of the reagents are crucial to ensure that the system can be preserved for a long time and maintain optimal activity. The entire RPA constant-temperature amplification enzyme system requires an activator to activate the reaction, so as to promote the work of the multi-enzyme system. For example, polyethylene glycol has the functions of improving the efficiency of the RPA amplification reaction, promoting the synthesis of DNA chains, and assisting in the balance and other functions between the recombinase and single-stranded protein. Magnesium ions can promote the binding of DNA polymerase and dNTP, and once they enter the system, the amplification reaction begins, and the synthesis of DNA chains is promoted. Currently, the more common commercial form of recombinase constant-temperature amplification technology is a freeze-dried + liquid reagent combination (semi-freeze-dried form), that is, only the temperature-sensitive reaction enzyme system is freeze-dried, and the activation system with high temperature tolerance is set as a liquid reagent, which is usually stored at -20°C. There are also all-liquid reagents, that is, all components are liquid, but require stringent storage conditions and need to be strictly stored at -20°C. However, both of the above two reagent forms have the problem of relatively complicated operation. During the use of semi-freeze-dried reagents, freeze-dried reconstitution agents/re-thawing agents and activators are required as necessary components of the reaction. There are three spotting steps, and the amount of activator added is 2-3µL/well. The requirements for experimental personnel and operating tools are high, the process is cumbersome, and it is easy to cause spotting errors. In the recombinase-mediated constant-temperature amplification system, the common re-thawing agents and activators in liquid form have an immediate activation effect on the system, that is, after the enzyme system and the activation system are mixed, the reaction begins immediately at room temperature. Therefore, in the early tests, the effect of simply mixing the whole system and then freeze-dried are not considerable, and the viscosity of polyethylene glycol is relatively high, which affects the homogeneity of the whole system.

Chinese patent CN110305975B discloses an RPA kit for rapid detection of chicken synoviae mycoplasma and use thereof. The kit includes: a reaction tube containing RPA freeze-dried particles, a reaction buffer, magnesium acetate, a pair of primers and a probe. In this kit, only the enzymes and other necessary items needed for amplification are freeze-dried for storage. During amplification, reaction buffer, primers, probes and templates still need to be added, and magnesium ions are added to initiate the reaction. The reaction steps are cumbersome, and the kit is inconvenient to transport and store.

Currently, a full-freeze-dried constant-temperature amplification system to be more sensitive, more specific and meeting the needs of rapid detection is needed.

### BRIEF DESCRIPTION OF THE INVENTION

As used herein, the singular forms "a", "an", and "the" include singular and plural referents, unless clearly otherwise indicated in the context. The recitation of numerical ranges by endpoints includes all numbers and fractions within the corresponding ranges, as well as the recited endpoints.

In view of the problems existing in a conventional technology, the present application provides a recombinase-mediated full-freeze-dried constant-temperature amplification system, and a preparation method therefor, and use thereof. The full-freeze-dried constant-temperature amplification system includes three parts, namely, freeze-dried powder of enzyme system, freeze-dried pellets of activation system, and freeze-dried pellets of a primer and probe. The three parts have different freeze-dried forms, which can improve the smoothness and homogeneity of the liquid, avoid the problem of high viscosity of the whole system and difficulty in dispersing and homogenizing during the freeze-dried process to result in unstable results, thereby realizing RPA full-freeze-dried and achieving one-step loading operation.

To achieve the above purpose, the technical solutions adopted by the present application are as follows.

The present application provides a full-freeze-dried constant-temperature amplification system including two parts:
a first part being an enzyme system, and a second part being a primer and probe system and an activation system; where the freeze-dried form of the enzyme system is freeze-dried powder, and the freeze-dried form of the primer and probe system and the activation system are freeze-dried pellets.

Preferably, the enzyme system includes: a protease mixture, dNTPs, Tris buffer, a kinase system, and mannosyl glycerate.

Preferably, the activation system includes: polyethylene glycol, trehalose, dithiothreitol, magnesium acetate or potassium acetate, and sodium chloride.

More preferably, the activation system includes polyethylene glycol, trehalose, dithiothreitol, magnesium acetate and sodium chloride.

Preferably, the protease mixture includes: a recombinase, a recombinant regulatory protein, a single-stranded DNA binding protein, a strand-displacing DNA polymerase and an RT enzyme; and the kinase system includes: ATP, creatine phosphokinase, and creatine phosphate.

Preferably, the activation system includes 1%-20% polyethylene glycol, 2%-10% trehalose, 1-15 mM dithiothreitol, 2-28 mM magnesium acetate, and 1-12mM sodium chloride, where the percentage concentration is the mass-volume concentration (w/v), where w is measured in g, and v is measured in mL.

More preferably, the activation system includes 20% g/mL polyethylene glycol, 2% g/mL trehalose, 3 mM dithiothreitol, 20 mM magnesium acetate, and 4 mM sodium chloride.

Preferably, the concentration of the primer is 100-800 nM; the concentration of the probe is 50-200 nM.

More preferably, the concentration of the primer is 400 nM; the concentration of the probe is 150 nM.

Particularly, the probe includes a fluorescent group and quenching group; where the fluorescent group includes FAM, HEX, ROX, JOE, VIC or CY5; and the quenching group includes BHQ1 or BHQ2.

Preferably, the fluorescent group includes FAM; and the quenching group includes BHQ1.

Preferably, the enzyme system includes a protease mixture, 10-60 mM dNTPs, 10-80 mM Tris buffer, a kinase system, and 1-5 mM mannosyl glycerate.

More preferably, the enzyme system includes a protease mixture, 40 mM dNTPs, 30 mM Tris buffer, a kinase system, and 1 mM mannosyl glycerate.

Preferably, the protease mixture includes 100-800 ng/µL recombinase, 200-800 ng/µL recombinant regulatory protein, 300-1000 ng/µL single-stranded DNA binding protein, 20-100 ng/µL strand-displacing DNA polymerase, and 160-200 U RT enzyme; the kinase system includes 1-10 mM ATP, 40-200 ng/µL creatine phosphokinase and 20-80 mM creatine phosphate.

More preferably, the protease mixture includes 200 ng/µL recombinase, 400 ng/µL recombinant regulatory protein, 300 ng/µL single-stranded DNA binding protein, 60 ng/µL strand-displacing DNA polymerase, and 160 U RT enzyme; the kinase system includes 3 mM ATP, 40 ng/µL creatine phosphokinase, and 40 mM creatine phosphokinase.

The present application also provides a full-freeze-dried constant-temperature amplification kit, including the above full-freeze-dried constant-temperature amplification system.

The present application also provides a method for preparing a full-freeze-dried constant-temperature amplification system, where the above enzyme system is freeze-dried to obtain a freeze-dried powder of the enzyme system, and the primer and probe system and activation system are mixed and freeze-dried to obtain freeze-dried pellets.

The present application also provides use of the above full-freeze-dried constant-temperature amplification system in recombinase polymerase amplification.

Compared with a conventional technology, the present application has the following beneficial effects.
(1) The full-freeze-dried constant-temperature amplification system according to the present application is easy to transport and operate, and only the template is needed to add to react on the detection instrument without other cumbersome steps.
(2) The full-freeze-dried constant-temperature amplification process according to the present application has low requirements on temperature, only a constant temperature reaction of 37-42°C is needed, and even lower requirements is needed for the heating module of reaction.
(3) The constant-temperature amplification process according to the present application has a short reaction time. Compared with the 1.5-3 hours of conventional PCR, this technology shortens the reaction time to 15-20 minutes, greatly improving the detection efficiency.
(4) The constant-temperature amplification system according to the present application has the advantages of both high sensitivity and high specificity.

### BRIEF DESCRIPTION OF THE DRAWINGS

Fig. 1 shows the detection results of influenza A virus in different Test Groups of the full-freeze-dried system.
Fig. 2 shows the detection results of human syncytial virus in different Test Groups of the full-freeze-dried system.
Fig. 3 shows the sensitivity of the detection of influenza A virus in the full-freeze-dried system.
Fig. 4 shows the sensitivity of the detection of human syncytial virus in the full-freeze-dried system.
Fig. 5 shows the results of the detection of 1500 copies/ml influenza A virus in the full-freeze-dried system, semi-freeze-dried system, and liquid system.
Fig. 6 shows the results of the detection of 1500 copies/mL human syncytial virus in the full-freeze-dried system, semi-lyophilized system, and liquid system.

### DETAILED DESCRIPTION OF THE INVENTION

The present application is described below through specific Examples to make the technical solutions of the present application easier to understand and grasp, but the present application is not limited thereto, and the described Examples are only part of the examples of the present application, rather than all of the examples.

The endpoints of the ranges and any values disclosed herein are not limited to the exact ranges or values, and these ranges or values should be understood to include values approximating these ranges or values. For numerical ranges, endpoint values of each range, an endpoint value of each range and an individual point value, and individual point values can be combined with each other to obtain one or more new numerical ranges, which should be regarded as particularly disclosed herein.

Based on the Examples in this application, all other examples obtained by ordinary technicians in this field without making any creative work should fall within the scope of protection of this application. The experimental methods described in the following Examples are conventional methods unless otherwise specified; the reagents and materials described are commercially available unless otherwise specified.

### Example 1

### 1. Preparation of constant-temperature amplification system

The system includes two parts:
(1) Constant-temperature amplification enzyme system includes protease mixture, 40 mM dNTPs, 30 mM Tris buffer, kinase system, 1 mM mannosyl glycerate; where the protease mixture includes: 200 ng/µL recombinase, 400 ng/µL recombinant regulatory protein, 300 ng/µL single-stranded DNA binding protein, 60 ng/µL strand-displacing DNA polymerase, and 160 U RT enzyme; the kinase system includes: 3 mM ATP, 40 ng/µL creatine phosphokinase, and 40 mM creatine phosphokinase.
(2) Primers and probes, and constant-temperature amplification activation system: including 400 nM primers, 150 nM probes, 20% g/mL polyethylene glycol, 2% g/mL trehalose, 3 mM dithiothreitol, 20 mM magnesium acetate, and 4 mM sodium chloride.

### 2. Grouping test on freeze-dried reagents

In order to test the effect of the full-freeze-dried system, the experimental freeze-dried Test Groups are set up as shown in Table 1. Test Group 1 and Test Group 2 are a combination of freeze-dried powder and freeze-dried pellets. The difference is that the systems in which the primer and probe are located are different. Test Group 1 consists of freeze-dried powder of the enzyme system and freeze-dried pellets of the activation system and primer and probe system, and Test Group 2 consists of freeze-dried powder of the enzyme system and primer and probes system, and freeze-dried pellets of the activation system. Both Test Group 3 and Test Group 4 mix the full constant-temperature amplification system and lyophilize the mixture, the difference is that Test Group 3 is freeze-dried powder, and Test Group 4 is freeze-dried pellets.

**Table 1: Freeze-dried tests for constant-temperature amplification system**

| Freeze-dried reagents | Test Group 1 | Test Group 2 | Test Group 3 | Test Group 4 |
|---|---|---|---|---|
| Freeze-dried powder | Enzyme system | Enzyme system; primers and probes | Enzyme system; activation system; primers and probes | / |
| Freeze-dried pellets | Activation system; primers and probes | Activation system | / | Enzyme system; activation system; primers and probes |

To further verify the detection effect, this test uses influenza A virus and human syncytial virus as test pathogens for exemplifying. The above-formulated reagents were freeze-dried in a -80°C freeze dryer under negative pressure to obtain freeze-dried powder and freeze-dried pellets of the reagents for subsequent testing.

The detection steps are as follows:
Step 1: Pseudoviruses of influenza A virus and human syncytial virus synthesized by General Biol (Anhui) Co., Ltd. were selected as templates, a magnetic bead kit (from Sansure Biotech Co., Ltd., catalogue number: S10015) was used to extract nucleic acids from the above pseudoviruses respectively, and the obtained nucleic acids were used as reaction templates.
Step 2: using 50 µL nucleic acids from pseudoviruses of influenza A virus and human syncytial virus as templates, the above freeze-dried reagents were re-thawed, then shaked and centrifuged to ensure that they are fully re-thawed before immediately loaded onto the instrument. The Hongshi fully automatic medical PCR analysis system (SLAN-96P) was used for detection, and the program was set to 39°C, 30s (fluorescence collection) for 40 cycles.

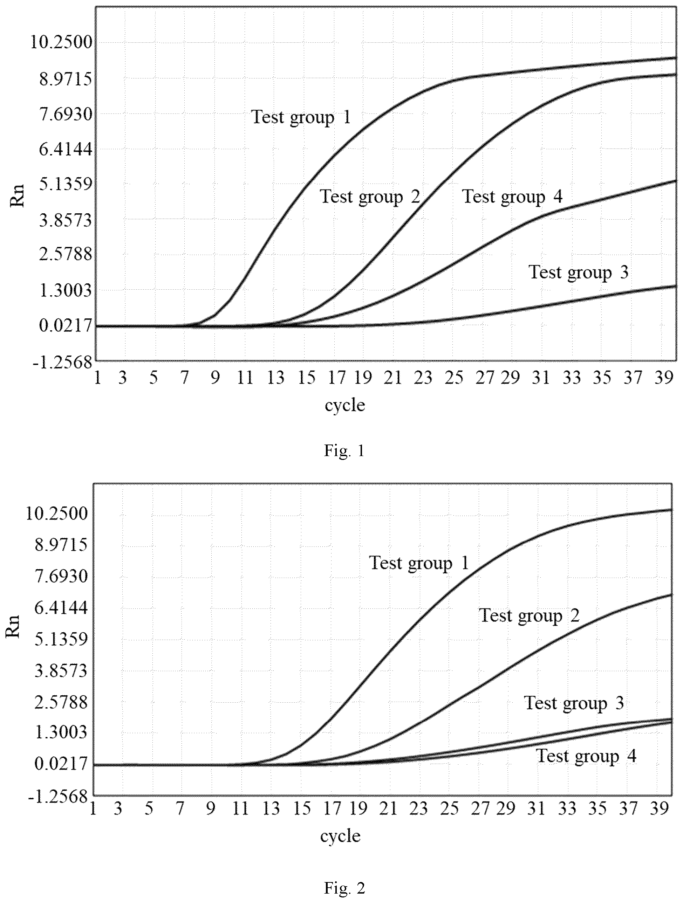

The results are shown in Figs. 1-2. By comparing the detection effects of different full-freeze-dried reagents, it was found in this example that the trends are consistent. The detection effects of Test Group 3 and Test Group 4 in the fully mixed freeze-dried system are not good, the fluorescence values are extremely low, and the peak time of the amplification curve is late. The effects of grouping freeze-dried are better. The fluorescence value of Example 1 is the highest, and the peak time of the amplification curve is the earliest. The peak time of the amplification curve of Test Group 2 is not significantly delayed, but the fluorescence value decreased significantly.

As shown in Test Group 1, the present application adjusted the distribution system of the freeze-dried reagent, prepared the enzyme system into freeze-dried powder, and mixed the activation system and the primer and probe system into freeze-dried pellets. The effect of the prepared freeze-dried reagent is better than that of Test Groups 2-4.

Therefore, the freeze-dried combination of Test Group 1 is preferred as Example 1 of the full lyophilized system solution.

### 3. Sensitivity detection

In order to further detect the sensitivity of the full-freeze-dried system, influenza A virus and human syncytial virus were used as test pathogens for exemplifying. The system was formulated and freeze-dried according to the scheme in Table 2 to obtain freeze-dried powder and freeze-dried pellets of the reagents for subsequent testing.

**Table 2: Formulation of full-freeze-dried constant-temperature amplification reaction system**

| Enzyme system for constant-temperature amplification (freeze-dried powder) | Final concentration of the system |
|---|---|
| Recombinase | 200ng/µL |
| Recombinant regulatory protein | 400ng/µL |
| Single-stranded DNA binding protein | 300 ng/µL |
| Strand-displacing DNA polymerase | 60ng/µL |
| RT enzyme | 160U |
| dNTP (s) | 40mM |
| Tris-buffer | 30mM |
| ATP | 3mM |
| Creatine phosphokinase | 40ng/µL |
| Creatine phosphate | 40mM |
| Mannosyl glycerate | 1mM |

| Activation system for constant-temperature amplification (freeze-dried pellets) | Final concentration of the system |
|---|---|
| Polyethylene glycol | 20% g/mL |
| Trehalose | 2% g/mL |
| Dithiothreitol | 3mM |
| Magnesium acetate | 20mM |
| Sodium chloride | 4mM |
| Primers | 400nM |
| Probes | 150nM |

Particularly, the forward and reverse primers and probe sequences for amplifying influenza A virus are as follows:
Forward primer: 5'-GGTCGAAACGTACGTTCTCTCTATCATTCCAT-3' (SEQ ID NO. 1);
Reverse primer: 5'-CAGTGAGCGAGGACTGCAGCGTAGACGGTT-3' (SEQ ID NO. 2);
Probe: 5'-TGCAGGGAAGAACGCAGATCTCGAGGCTCTCATGGAGTGGATAAAG-3' (SEQ ID NO. 3);

The modified influenza A virus probe is as follows:
TGCAGGGAAGAACGCAGATCTCGAGGCTC[FAM-dT]C[THF][BHQ-dT]GGAGTGGATAAAG-C3 Spacer, where the 30th base T is labeled with the fluorescent group FAM, the 33rd base T is labeled with the quencher group BHQ1, the 32nd base A of the probe sequence is replaced with tetrahydrofuran, and the 3' end is labeled with C3-spacer.

The forward and reverse primers and probe sequences for amplifying human syncytial virus are as follows:
Forward primer: 5'-CAACACCCTGACAAAATCAACAATATAGTAACAA-3' (SEQ ID NO. 4);
Reverse primer 5'-GCAAGTCTGCTGGCACAGATGACTGGAACATAG-3' (SEQ ID NO. 5); Probe:
5'-TTCAGTACAATGTTCTAGAAAAAGATGATGATCCTGCATCACTAACAATATG-3' (SEQ ID NO. 6);

The modified human syncytial virus probe is as follows:
TTCAGTACAATGTTCTAGAAAAAGATGATGATCC[FAM-dT]G[THF]A[BHQ-dT]CACTAACAATAT-C3 Spacer, where the 35th base T is labeled with the fluorescent group FAM, the 39th base T is labeled with the quenching group BHQ1, the 37th base C of the probe sequence is replaced with tetrahydrofuran, and the 3' end is labeled with C3-spacer.

The sensitivity detection steps are as follows:
Step 1: Pseudoviruses of influenza A virus and human syncytial virus synthesized by General Biol (Anhui) Co., Ltd. were selected as templates, a magnetic bead kit (from Sansure Biotech Co., Ltd., catalogue number: S10015) was used to extract nucleic acids from the above pseudoviruses respectively, and the obtained nucleic acids were diluted with deionized water into corresponding gradients (1500cp/mL, 900cp/mL, 300cp/mL, and 150cp/mL) as reaction templates.
Step 2: using 50 µL nucleic acids from pseudoviruses of influenza A virus and human syncytial virus as templates, the above freeze-dried reagents were re-thawed, then shaked and centrifuged to ensure that they are fully re-thawed before immediately loaded onto the instrument. The Hongshi fully automatic medical PCR analysis system (SLAN-96P) was used for detection, and the program was set to 39°C, 30s (fluorescence collection) for 40 cycles.

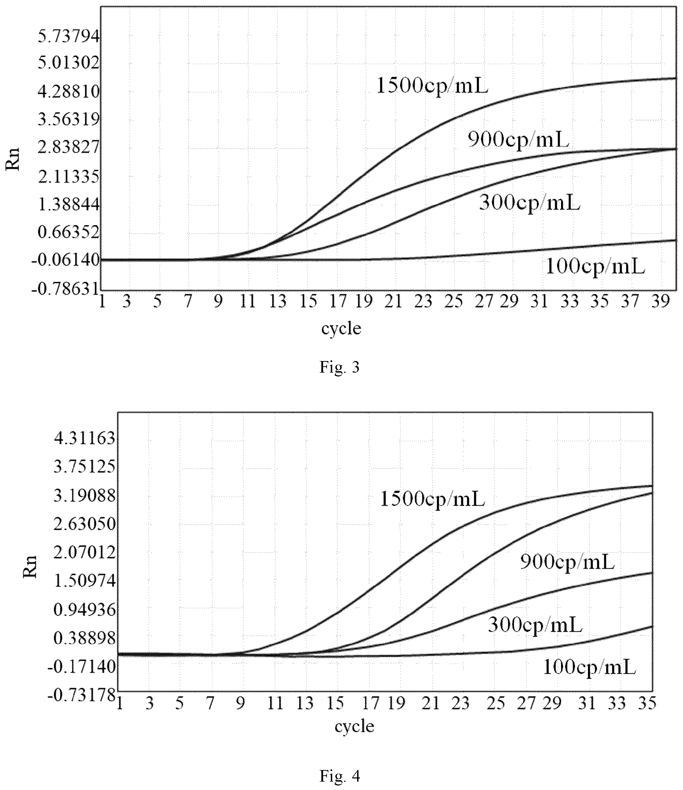

The results are shown in Figs. 3-4. The sensitivity detection effect of the fully mixed freeze-dried system is good. It can be seen from Figs. 3-4 that, 300 copies/ml of nucleic acids from pseudoviruses of influenza A virus and human syncytial virus are detected in Example 1. For nucleic acids at a medium and high concentration of 1500 copies/ml, the peak starting time of Example 1 is about 5 minutes, and the detection is fast.

The specific detection results of the sensitivity are shown in Table 3, and Example 1 meets the requirements.

**Table 3: Sensitivity detection results of influenza A virus and human syncytial virus in full-freeze-dried constant-temperature amplification system**

| Concentration | Detection rate of influenza A virus | Detection rate of human syncytial virus |
|---|---|---|
| 1500 copies/mL | 100% (20/20) | 100% (20/20) |
| 900 copies/mL | 100% (20/20) | 100% (20/20) |
| 300 copies/mL | 100% (20/20) | 100% (20/20) |
| 150 copies/mL | 80% (16/20) | 70% (14/20) |

### Comparative Example 1: Semi-freeze-dried System

Compared with Example 1, the activation system of the freeze-dried pellets is presented in the form of a liquid reagent to form a composition as a constant-temperature amplification reagent in which only the enzyme system is lyophilized into a freeze-dried powder. The specific formulation is shown in Table 4:

**Table 4: The schedule for the formulation of a constant-temperature amplification enzyme system**

| Components of the constant-temperature amplification enzyme system | Final concentration of the system |
|---|---|
| Recombinase | 200ng/µL |
| Recombinant regulatory protein | 400ng/µL |
| Single-stranded DNA binding protein | 300 ng/µL |
| Strand-displacing DNA polymerase | 60ng/µL |
| RT enzyme | 160U |
| dNTP (s) | 40mM |
| Tris-buffer | 30mM |
| ATP | 3mM |
| Creatine phosphokinase | 40ng/µL |
| Creatine phosphate | 40mM |
| Mannosyl glycerate | 1mM |

Step 1: Pseudoviruses of influenza A virus and human syncytial virus synthesized by General Biol (Anhui) Co., Ltd. were selected as templates, a magnetic bead kit (from Sansure Biotech Co., Ltd., catalogue number: S10015) was used to extract nucleic acids from the above pseudoviruses respectively, and the obtained nucleic acids were diluted with deionized water into corresponding gradients (1500cp/mL, 900cp/mL, 300cp/mL, 150cp/mL) as reaction templates.

Step 2: After formulating the enzyme system according to Table 4, it was freeze-dried for later use.

Step 3: 12.5 µL of re-thawing agent (20% g/mL polyethylene glycol, 2% g/mL trehalose, 3 mM dithiothreitol) and 1 µL of primer and probe (400 nM primers, and 150 nM probes) were used to fully re-thaw and mix the enzyme system, then adding 2.5 µL of activator (20 mM magnesium acetate, 4 mM sodium chloride) into the cap of the eight-tube; and the template was used to make up to 50 µL.

Step 4: the above mixture was mixed well to perform centrifugation, then placed on the Hongshi fully automatic medical PCR analysis system SLAN-96P; the program was set to 39°C, 30s (fluorescence collection) for 40 cycles. After obtaining the amplification plots, analysis is performed.

### Comparative Example 2: Liquid System

Compared with Example 1, the freeze-dried step was eliminated, and the constant-temperature amplification reagent was presented in the form of a liquid reagent. The template was used to make up to 50 µL, and other experimental conditions remained unchanged.

Step 1: Pseudoviruses of influenza A virus and human syncytial virus synthesized by General Biol (Anhui) Co., Ltd. were selected as templates, a magnetic bead kit (from Sansure Biotech Co., Ltd., catalogue number: S10015) was used to extract nucleic acids from the above pseudoviruses respectively, and the obtained nucleic acids were diluted with deionized water into corresponding gradients (1500cp/mL, 900cp/mL, 300cp/mL, 150cp/mL) as reaction templates.

Step 2: After formulating the enzyme system according to Table 4, 12.5 µL of re-thawing agent (20% g/mL polyethylene glycol, 2% g/mL trehalose, 3 mM dithiothreitol) and 1 µL of primer and probe (400 nM primers, and 150 nM probes) were used to fully mix the enzyme system, then adding 2.5 µL of activator (20 mM magnesium acetate, 4 mM sodium chloride) into the cap of the eight-tube; and the template was used to make up to 50 µL.

Step 3: the above mixture was mixed well to perform centrifugation, then placing on the Hongshi fully automatic medical PCR analysis system SLAN-96P; the program was set to 39°C, 30s (fluorescence collection) for 40 cycles. After obtaining the amplification plots, analysis is performed.

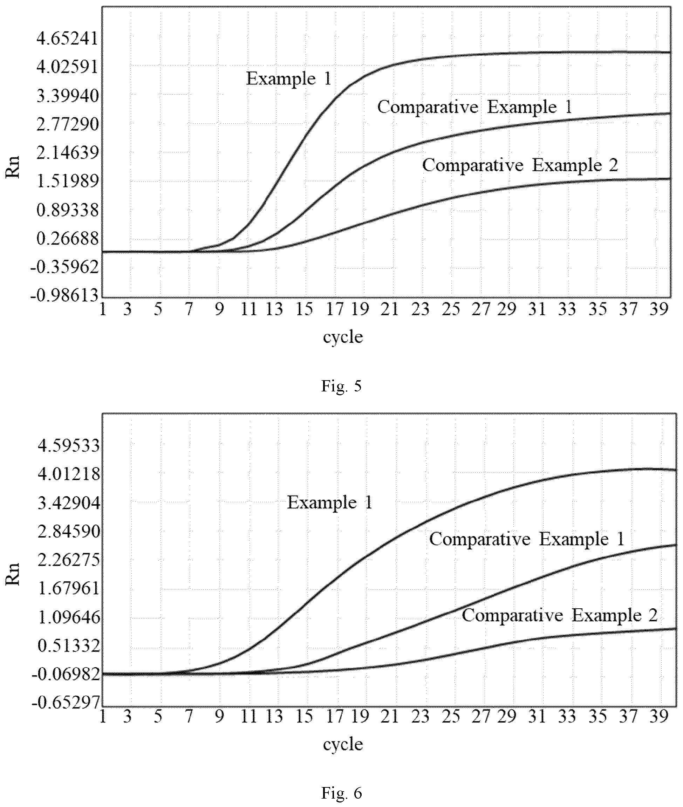

The sensitivity results of Example 1, Comparative Example 1 and Comparative Example 2 are as follows:
It can be seen from the amplification plots of 1500 copies/ml (Figs. 5-6) that, Example 1 has a better detection effect, a stronger fluorescence intensity, and an earlier peak starting time; while the peak starting time of Comparative Example 1 is relatively late, and the fluorescence value is lower. The detection effect of Comparative Example 2 is poor, and templates below 1500 copies/ml cannot be detected. The specific detection results of the sensitivity are shown in Table 5.

**Table 5: Sensitivity detection results of the constant-temperature amplification system for influenza A virus and human syncytial virus**

| | Example 1 | | Comparative Example 1 | | Comparative Example 2 | |
|---|---|---|---|---|---|---|
| Concentration | Detection rate of influenza A virus | Detection rate of human syncytial virus | Detection rate of influenza A virus | Detection rate of human syncytial virus | Detection rate of influenza A virus | Detection rate of human syncytial virus |
| 1500 copies/mL | 100% (20/20) | 100% (20/20) | 100% (20/20) | 100% (20/20) | 100% (20/20) | 100% (20/20) |
| 900 copies/mL | 100% (20/20) | 100% (20/20) | 100% (20/20) | 100% (20/20) | 80% (16/20) | 60% (12/20) |
| 300 copies/mL | 100% (20/20) | 100% (20/20) | 85% (17/20) | 95% (19/20) | 55% (11/20) | 40% (8/20) |
| 150 copies/mL | 80% (16/20) | 70% (14/20) | 65% (13/20) | 75% (15/20) | 25% (5/20) | 15% (3/20) |

Example 1 can stably detect pseudovirus samples of influenza A virus and human syncytial virus with the concentration of 300 copies/ml. The detection rates of Comparative Example 1 for 300 copies/ml pseudovirus sample of influenza A virus and human syncytial virus are 85% and 95%, respectively. The detection rates of Comparative Example 2 for 300 copies/ml pseudovirus sample of influenza A virus and human syncytial virus are 55% and 40%, respectively. The above results show that, the sensitivity of the full-freeze-dried system is better than that of the semi-freeze-dried system, and is significantly better than that of the liquid system.

Finally, it should be noted that the above content is only used to illustrate the technical solutions of the present application, rather than to limit the protection scope of the present application. Simple modifications or equivalent substitutions to the technical solutions of the present application by a person skilled in this art do not deviate from the essence and scope of the technical solutions of the present application.

## Claims

1. A full-freeze-dried constant-temperature amplification system, **characterized by** comprising two parts:
a first part being an enzyme system, and a second part being a primer and probe system and an activation system; wherein the freeze-dried form of the enzyme system is freeze-dried powder, and the freeze-dried form of the primer and probe system and the activation system are freeze-dried pellets.

2. The full-freeze-dried constant-temperature amplification system according to claim 1, **characterized in that** the enzyme system comprises: a protease mixture, dNTPs, Tris buffer, a kinase system, and mannosyl glycerate; andthe activation system comprises: polyethylene glycol, trehalose, dithiothreitol, magnesium acetate or potassium acetate, and sodium chloride.

3. The full-freeze-dried constant-temperature amplification system according to claim 2, **characterized in that** the protease mixture comprises: a recombinase, a recombinant regulatory protein, a single-stranded DNA binding protein, a strand-displacing DNA polymerase and an RT enzyme; and the kinase system comprises: ATP, creatine phosphokinase, and creatine phosphate.

4. The full-freeze-dried constant-temperature amplification system according to claim 1, **characterized in that** the concentration of the primer is 100-800 nM; andthe concentration of the probe is 50-200 nM.

5. The full-freeze-dried constant-temperature amplification system according to claim 2, **characterized in that** the enzyme system comprises: a protease mixture, 10-60 mM dNTPs, 10-80 mM Tris buffer, a kinase system, and 1-5 mM mannosyl glycerate.

6. The full-freeze-dried constant-temperature amplification system according to claim 2, **characterized in that** the activation system comprises: 1%-20% g/mL polyethylene glycol, 2%-10% g/mL trehalose, 1-15 mM dithiothreitol, 2-28 mM magnesium acetate, and 1-12 mM sodium chloride.

7. The full-freeze-dried constant-temperature amplification system according to claim 3, **characterized in that** the protease mixture comprises: 100-800 ng/µL recombinase, 200-800 ng/µL recombinant regulatory protein, 300-1000 ng/µL single-stranded DNA binding protein, 20-100 ng/µL strand-displacing DNA polymerase, and 160-200 U RT enzyme; the kinase system comprises: 1-10 mM ATP, 40-200 ng/µL creatine phosphokinase, and 20-80 mM phosphocreatine.

8. A full-freeze-dried constant-temperature amplification kit, **characterized by** comprising the full-freeze-dried constant-temperature amplification system according to any one of claims 1 to 7.

9. A method for preparing a full-freeze-dried constant-temperature amplification system, **characterized in that** the enzyme system according to any one of claims 1 to 7 is freeze-dried to obtain a freeze-dried powder of the enzyme system, and the primer and probe system and activation system are mixed and freeze-dried to obtain a freeze-dried pellet.

10. Use of the full-freeze-dried constant-temperature amplification system according to any one of claims 1 to 7 in recombinase polymerase amplification.
